(19) Europäisches Patentamt
European Patent Office
Office européen des brevets

(11) Publication number: **0 045 717**
**B1**

(12) # EUROPEAN PATENT SPECIFICATION

(45) Date of publication of patent specification: **25.09.85**

(51) Int. Cl.$^4$: **C 07 D 501/28,**
C 07 D 501/18, C 07 D 501/36

(21) Application number: **81810288.1**

(22) Date of filing: **16.07.81**

(54) **New cephalosporin derivatives, their production and their use.**

(30) Priority: **01.08.80 AT 3999/80**
**26.11.80 AT 5767/80**
**28.11.80 AT 5819/80**

(43) Date of publication of application:
**10.02.82 Bulletin 82/06**

(45) Publication of the grant of the patent:
**25.09.85 Bulletin 85/39**

(84) Designated Contracting States:
**AT BE CH DE FR GB IT LI LU NL SE**

(56) References cited:
**DE-A-3 002 659**

(73) Proprietor: **BIOCHEMIE Gesellschaft m.b.H.**
**A-6250 Kundl Tirol (AT)**

(72) Inventor: **Ascher, Gerd, Dr.**
**18 Bahnhofstrasse**
**A-6250 Kundl 506 (AT)**
Inventor: **Prager, Bernhard Christian, Dr.**
**No. 264**
**A-6250 Kundl (AT)**

(74) Representative: **Norris, Allen Edwin et al**
**SANDOZ AG Patentabteilung**
**CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 717 B1

**Description**

7-Aminocephalosporanic acid derivatives are key intermediates for the production of a wide variety of semi-synthetic cephalosporin antibiotics. One method for their production involves the deacylation of corresponding 7-acylaminocephalosporanic acids, which may have been produced directly by fermentation, or may be derivatives of fermentation products. A well-known deacylation method involves the so-called iminohalide/iminoether route, in which the carbonyl group in the 7-amido linkage is converted in to an iminohalide by treatment with a halogenating agent such as phosphorous pentachloride; the amino halide is then converted into an imino ether with an alcohol and the imino ether is readily split by hydrolysis to form the free 7-aminocephalosporanic acid. This process as applied to cephalosporin C has been described for example in British Patent 1,041,985.

In an earlier application (DOS 3,002,659), we have described a highly advantageous improvement in the method for converting Cephalosporin C to 7—ACA which involves the initial formation of Cephalosporin C derivatives of formula A;

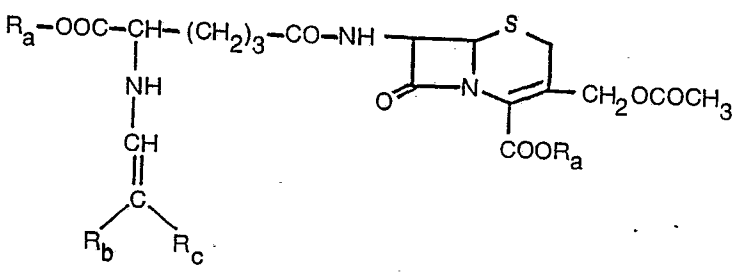

A

in which the radicals $R_a$, which may be the same or different, each signifies hydrogen or the residue of an ester grouping, and $R_b$ and $R_c$, which may be the same or different each signifies hydrogen, nitro, cyano or lower alkoxycarbonyl.

The derivatives of formula A may readily be deacylated, for example by the imino halide/imino ether route, to form 7—ACA or salts or esters thereof. One major advantage of this method is that the formation of the compounds of formula A may be accomplished readily in Cephalosporin C culture filtrates thus avoiding the need to isolate Cephalosporin C which is laborious. A further advantage is that the reagents employed to produce the derivative of formula A are stable to aqueous hydrolysis and need not therefore be employed in great excess, in contrast to other known processes in which the amino group of the cephalosporin side is protected prior to the deacylation step, for example by reaction with acid chlorides. The avoidance of such excesses minimises side reactions and thus increases yields. We designate the derivatives of formula A as polar olefines and, therefor, the method is designated the Polar Olefine Method.

We have now surprisingly found that other polar olefines may be used in the method of converting Cephalosporin C to 7—ACA. We have also surprisingly found that the polar olefine method has quite general applicability not only to Cephalosporin C itself but to other known cephalosporins having an aminoadipoyl side chain, for example the cephamycins. Finally, we have discovered that the polar olefins are well suited, for example sufficiently stable, for further derivatisation, in particular at the 3-substituent, more particularly by thiolation, prior to the deacylation step.

In one aspect, therefore, the present invention concerns a novel class of polar olefines of formula I,

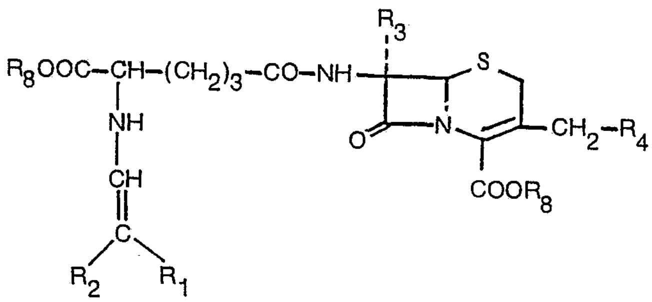

I

in which either $R_4$ is a group $R_5$—CO—O, in which $R_5$ is lower alkyl, amino, mono- or di-(lower alkyl) amino, or α-methoxy-β-(*p*-hydroxyphenyl) vinyl, in which the hydroxy group may be esterified with sulphuric acid, or $R_4$ is a group $R_6$—S—, in which $R_6$ is a heterocycle, $R_3$ is hydrogen or methoxy, $R_1$ and $R_2$, wich may be the same or different, are either lower alkanoyl when $R_4$ is $CH_3$—CO—O; or hydrogen, nitro, cyano, $(C_{1-4})$

alkanoyl or ($C_{1\text{-}4}$) alkoxycarbonyl, when $R_4$ is other than $CH_3—CO—O—$, and the radicals $R_8$, which may be the same or different, each signifies hydrogen or the residue of an ester grouping, and salts of compounds in which one or both of the radicals $R_8$ represents hydrogen, and we claim compounds of formula I wherein $R_4$ is $R_6—S—$.

Compounds of formula I may be produced by processes a) and b).

In process a) a compound of formula Ia,

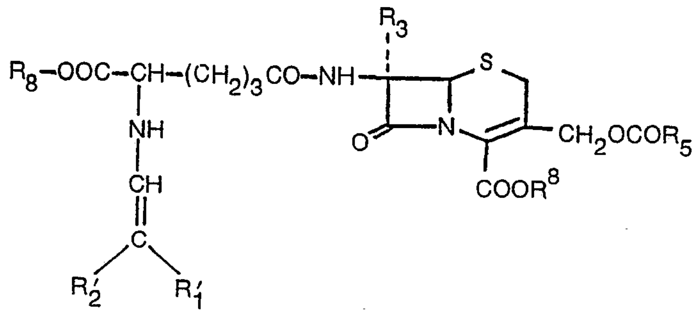

Ia

in which $R_3$, $R_5$ and $R_8$ are as defined above, and $R_2'$ and $R_1'$, which may be the same or different, each signifies either lower alkanoyl when $R_5$ is $CH_3—$; or hydrogen, nitro, cyano, lower alkanoyl or lower alkoxycarbonyl when $R_5$ is other than $CH_3—$, may be produced by reacting a compound of formula II,

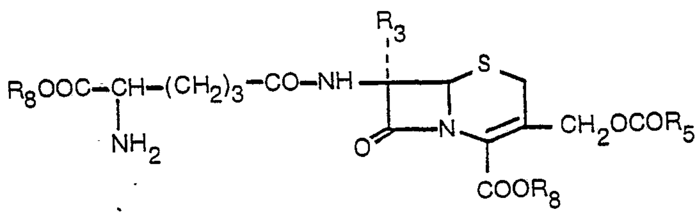

II

in which $R_3$, $R_5$ and $R_8$ are as defined above, with a compound of formula III,

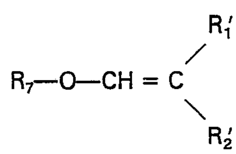

III

in which $R_1'$ and $R_2'$ are as defined above, and $R_7$ is hydrogen or lower alkyl.

In process b) a compound of the formula Ib,

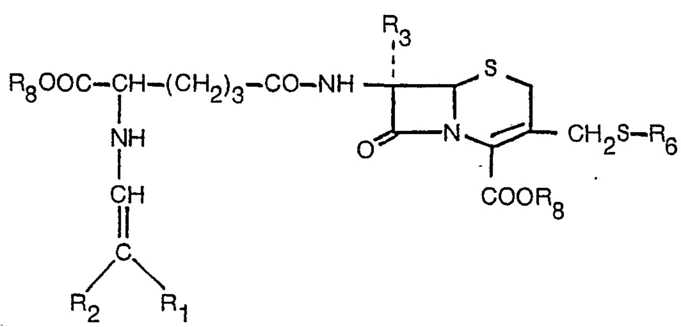

Ib

in which $R_1$, $R_2$, $R_3$, $R_6$ and $R_8$ are as defined above, may be produced by reacting a compound of formula Ic,

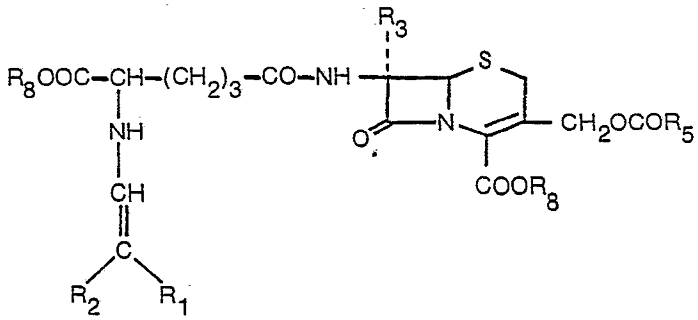

Ic

in which $R_1$, $R_2$, $R_3$, $R_5$ and $R_8$ are as defined above, with a compound of formula IV,

$$HS—R_6 \qquad IV$$

in which $R_6$ is as defined above.

Process b) forms part of the present invention.

Process a) is suitably effected in an inert solvent or solvent mixture, e.g. in an aqueous medium or in a mixture of water and a water-miscible solvent, such as an alkanol, e.g. ethanol, or acetone, preferably in water. The reaction is conveniently effected at a temperature of from 5° to 50°C, preferably 10° to 40°C, in particular at room temperature. The compound of formula II may conveniently be employed, when one or both of the radicals $R_8$ are hydrogen, in free acid form or in salt form, for example alkali metal salt form, e.g. in mono- or di-sodium salt form.

Process b) is suitably effected in aqueous solution, preferably working under neutral or weakly acid or alkaline conditions, more particularly from pH 4 to 9, in particular pH 6.5. The aqueous medium may suitably contain a hydrophilic, organic solvent, for example acetone, and the reaction may conveniently be effected at a temperature of from 20°C to 100°C, preferably 50° to 70°.

Process b) may alternatively be effected in an inert organic solvent or solvent mixture; for example aliphatic or aromatic hydrocarbons, amides, ethers, ketones, carboxylic acid esters, aliphatic or aromatic halohydrocarbons, nitro-hydrocarbons and nitriles, come into question. Specific examples of suitable solvents are toulene, xylene, dioxane, methyl isobutyl ketone, cyclohexanone, butyl acetate, diethyl carbonate, 1,2-dichloroethane, carbon tetrachloride, chloroform, 1,1,1- or 1,1,2-trichloroethane, 1,1,2,2-tetrachloroethane, chlorobenzene, dichlorobenzene, nitromethane, nitroethane, nitrobenzene, propionitrile, and butyronitrile, preferably 1,1,2-trichloroethane, 1,1,2,2-tetrachloroethane, methyl isobutyl ketone, nitrobenzene, nitromethane or nitroethane. Preferably the solvent is chosen such that the reactants are soluble at the desired reaction temperature. The process is suitably effected at 50° to 180°C.

Alternatively, process b) may be effected without a solvent, the two reactants then being well mixed and heated to the reaction temperature, in most cases a clear melt (or solution) then being formed.

The molecular ratio of the reactants is not critical but generally the compound of formula IV is employed in equimolar or slightly excess quantities; preferably 1 to 3, more preferably 1 to 1.5 molar equivalents of the compound IV are employed per molar equivalent of the compound Ic. The reactants may be employed in free form or in the form of salts, for example alkali metal salts, e.g. sodium or potassium salts.

The resulting compounds of formula I may, if desired, be isolated and purified in conventional manner. Where required, compounds of formula I in which one or both of $R_8$ are hydrogen may be converted into salt forms, e.g. alkali metal, such as potassium or sodium salt forms; in conventional manner, or *vice versa.*

In the compounds of formula I, the ester grouping(s) formed by the radical(s) $R_8$ may be any conventional ester protecting group employed in cephalosporin chemistry, in particular in deacylation processes for the production of 7—ACA or derivatives thereof. Preferred esters include the trialkylsilyl, dialkylchlorosilyl, benzhydryl and nitrobenzyl esters. The groups $R_8$, and the groups $R_1$ and $R_2$, are suitably the same. Alternatively, one may be hydrogen and the other, for example, lower alkanoyl. As used herein, the term "lower" when applied to alkyl groups or groups containing alkyl groups, means $C_{1-4}$. e.g. $C_{1-2}$, particularly $C_1$.

The compounds of formula I are, as indicated, useful as intermediates for the production of 7—ACA derivatives of formula V,

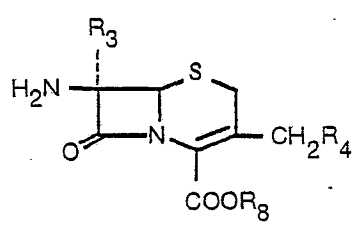

V

in which $R_3$, $R_4$ and $R_8$ are as defined above, and salts of the compounds in which $R_8$ is hydrogen.

The compounds I may be converted into compounds V by well-known deacylation methods such as the iminohalide/iminoether route described above. The compounds of formula V are themselves, of course, key starting materials for a wide range of known, highly active cephalosporin antibiotics.

A further aspect of the present invention is a process for converting a compound of formula VI,

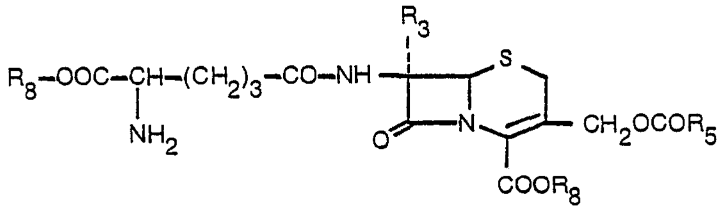

VI

in which $R_3$, $R_5$ and $R_8$ are as defined above, into a compound of formula V, stated above and wherein $R_4$ is $R_6$—S— with $R_6$ being as defined above, which comprises protecting the free amino group in the 7-aminoadipoyl side chain of the compound of formula VI to obtain a compound of formula Ia (i.e. a compound of formula Ic wherein $R_1$ and $R_2$ may be the same or different and each signifies $(C_{1-4})$ alkanoyl when $R_5$ is —$CH_3$ or hydrogen, nitro, cyano, $(C_{1-4})$ alkanoyl or $(C_{1-4})$ alkoxycarbonyl when $R_5$ is other than $CH_3$—), thiolating at the 3-position and deacylating at the 7 amino group.

In the compounds of formula I, the groups $R_1$ and $R_2$ may be alkanoyl, e.g. acetyl. Alternatively, they may be hydrogen. Alternatively, they may be nitro. Alternatively, they may be cyano. Finally, they may be alkoxycarbonyl.

The preferred significances for $R_4$ will of course be dictated by the desired product of formula V, or, ultimately, the desired cephalosporin antibiotic to be produced therefrom. These are of course known products. When $R_4$ is —S—$R_6$, the heterocycle $R_6$ may for example be: pyrrole, oxazole, isoxazole, thiazole, isothiazole, pyrazole, imidazole, 1,2,3-oxadiazole, 1,2,4-oxadiazole, 1,2,5-oxadiazole, 1,2,3-thiadiazole, 1,2,4-thiadiazole, 1,2,5-thiadiazole, 1,3,4-thiadiazole, 1,2,3-triazole, 1,2,4-triazole, oxatriazole, tetrazole, pyridine, pyridazine, pyrimidine, pyrazine, 1,2,4,5-tetrazine, benzthiazole, benzimidazole, purine, pteridine, quinoline, quinazoline, tetrazolo-[1,5,6]pyridazine. The heterocycle may be unsubstituted. Alternatively, it may be mono- or poly-substituted for example by lower alkyl, lower alkenyl, lower alkynyl, benzyl, aryl (for example phenyl or substituted phenyl), halogen, trifluoromethyl, amino, carboxy, carbamoyl, carbalkoxy, lower aminoalkyl, lower carboxyalkyl, lower carbalkoxy alkyl, or lower carbamoylalkyl.

In particular the heterocycle may suitably be 2-methyl-3-pyrazole, 2-methyl-3-imidazole, 1-methyl-1,2,3-triazole-5, 3,5-dimethyl-1,2,3-triazole-4, 4-methyl-1,2,4-triazole-5, 3-methyl-1,2,3-triazole-4, 5-methyl-1,2,3-oxadiazole-4, 1,2,4-oxadiazole-3, 3-methyl-1,2,4-oxadiazole-5, 3-methyl-1,2,5-oxadiazole-4, 3-methyl-1,2,4-thiadiazole-5, 1,3,4-thiadiazole-5, 2-methyl-1,3,4-thiadiazole-5, 2-(N-methylacetamido)-1,3,4-thiadiazole-5, 5-tetrazole, 1-methyltetrazole-5, 1-carboxymethyltetrazole-5, 1-(2-dimethylaminoethyl)tetrazole-5, 1,2,3,4-oxatriazole-5, 2-pyridine, 2-pyrimidine, 4-pyridine, 3-pyridazine, 2-pyrazine, 2-carbamoylmethyl-1,3,4-thiadiazole, 2-carbamoylmethyl-1,3,4-oxadiazole-5, 2-carbamoylmethyl-1,3,4-oxadiazole-5, 1-carbamoylmethyl-1,2,3,4-tetrazole-5, protected 1-(β-aminoethyl)tetrazole-5, 1-(β-carboxyethyl)tetrazole-5, 2,5-dihydro-6-hydroxy-2-methyl-5-oxo-1,2,4-triazine-3, 4,5-dihydro-6-hydroxy-4-methyl-5-oxo-1,2,4-triazine-3, 1,3,5-triazine-2, benzimidazole-2, benzothiazole-2, benzoxazole-2 and tetrazolo-[1,5,6]pyridazine-6.

The compounds of formula IV, for use in process b), are known or may be produced in conventional manner from available starting materials, for example as described in Heterocyclic Compounds, Robert C. Elderfield (John Wiley & Sons, N.Y.) or The Chemistry of Heterocyclic Compounds, Weissberger et al. (John Wiley & Sons, N.Y.). The compound dihydro-1,2,4-triazin-3-thiol may be produced analogously to the method described by K. H. Ongania (Dissertation, Innsbruck 1972). When the compound of formula IV for use in process b) contains a free amino group, this should preferably be protected prior to the reaction, the protecting group in the resultant product being removable in conventional manner.

The following examples, in which all temperatures are in degrees Centrigrade, illustrate the invention.

Example 1

N-(2,2-Diacetylvinyl)cephalosporin C (Process a)

9.5 g of Cephalosporin C monosodium salt dihydrate are dissolved in water and the pH is adjusted to 8 by addition of 2N NaOH. 4 g of 3-ethoxymethylenepentan-2,4-dione are added, whereupon the pH value immediately begins to drop. By continuous addition of 2N NaOH the pH value is held at about 8. After about 30 minutes the pH value of the solution is adjusted to 2 and the reaction mixture is extracted with n-butanol. The organic phase is dried and the solvent is evaporated off. The residue is rubbed with diisopropylether and dried *in vacuo* to obtain the heading compound, m.p. >100° (decomp.).

Example 2

N-(2,2-Diacetylvinyl)cephalosporin C monosodium salt (Process a)

1 litre of a conventionally obtained culture filtrate of a Cephalosporin C fermentation (content of active

substance 13 g/l) is adjusted to pH 8 by addition of 2N NaOH. 15 ml of 3-ethoxymethylene-pentan-2,4-dione are added, with stirring, whereby the pH value is maintained at 8 by continuous addition of NaOH. After about 30 minutes there is no further fall in pH value even without addition of NaOH. The pH value is then adjusted to 2 and the mixture is extracted with *n*-butanol. The organic phase is substantially evaporated and the residue is taken up in 300 ml of acetone. The monosodium salt is precipitated out by addition of 5 g of sodiumethylhexoate (dissolved in 50 ml of acetone). The salt is isolated by filtration and dried *in vacuo.* M.P. >160—179 (decomp.).

Example 3

7-[N-(2,2-diethoxycarbonyl)vinyl]adipinamido-3-[2,5-dihydro-6-hydroxy-5-oxo-2-methyl-1,2,4-triazin-3-yl)thionmethyl]ceph-3-em-4-carboxylic acid (Process b)

11.7 g of N-[(2,2-diethoxycarbonyl)vinyl]-cephalosporin C and 3.5 g of 2,5-dihydro-6-hydroxy-5-oxo-2-methyl-1,2,4-triazin-3-thiol are suspended in 100 ml of water. The pH value of the mixture is adjusted to 6.5 by addition of sodium bicarbonate, whereupon a clear solution results. The solution is warmed to 60° and stirred at this temperature for 6 hours whereupon the pH value remains at 6.5. The mixture is then cooled to room temperature and its pH value is adjusted to 2 by addition of semi-concentrated sulphuric acid. The resulting partially oily precipitate is extracted with a mixture of ethyl acetate and acetone (5:1) and the extract is freed of solvent and the residue digested with acetonitrile. The resulting solid material is isolated and dried at 40° *in vacuo* to obtain the heading compound as a light beige, substantially pure, powder. M.P. >165° (decomp.). 1H—NMR (MeOH-$d_4$): $\sigma$ = 9.30 (q, 1H, —CO—N<u>H</u>—); 8,10 (d, 1H, >N—C<u>H</u> =); 5.70 (1H, $H_7$); 5.00 (1H, $H_6$); 4.20 (4H, O—C<u>$H_2$</u>—); 3.70 (s, 3H, NC<u>$H_3$</u>); 3.65 (q, 2H, S—C<u>$H_2$</u>); 1.30 (2t, 6H, —$CH_2$—C<u>$H_3$</u>).

The heading compound may also be isolated in the form of its sodium salt by taking up the evaporation residue in acetone and adding a solution of sodium ethyl hexoate in acetone or a methanolic solution of sodium acetate, filtering and drying at 50°.

Example 4

7-[N-(2,2-diethoxycarbonyl)vinyl]adipinamido-3-[(1-methyl-1,2,3,4-tetrazol-5-yl)thiomethyl]-ceph-3-em-4-carboxylic acid, sodium salt (Process b)

5.85 g of N-[(2,2-diethoxycarbonyl)vinyl]-cephalosporin C together with 1.35 g of 1-methyl-5-mercaptotetrazole are suspended in 70 ml of water. The pH value is adjusted to 6.5 with solid sodium bicarbonate whereupon a clear solution results. This solution is stirred for 5 hours at 60-70° and then cooled to room temperature. The pH is adjusted to 5 with dilute sulphuric acid and the acid phase, from which an oily precipitate separates out, is extracted with a mixture of ethyl acetate/acetone (5:1). The extract is washed with saturated sodium chloride solution and then evaporated. The residue is taken up in 70 ml of acetone, filtered from undissolved material and mixed with a solution of sodium ethyl hexoate in acetone whereupon a voluminous precipitate forms. The precipitate is filtered, washed with acetone and dried at 50° *in vacuo* to obtain the heading compound in the form of a substantially pure, light beige-yellow powder, m.p. >175° (decomp). 1H—NHR (MeOH-$d_6$): = 9.20 (q, 1H, =C—N—<u>H</u>, J = 8 Hz, J = 16 Hz); 8,00 (d, 1H, =C—<u>H</u>, J = 16 Hz); 5.50 (d, 1H, $H_7$, J = 5Hz); 4.95 (d, 1H, $H_6$, J = 5 Hz); 4.20 (m, 7H, 2x-<u>O$CH_2$</u>—, $C_3$—<u>$CH_2$</u>—S—, >N—<u>C</u>—COOH); 3.98 (s, 3H, N—C<u>$H_3$</u>); 3.60 (q, 2H, S—C<u>$H_2$</u>—, J = 18 Hz); 2.40-1.60 (2m, 6H, —C<u>$H_2$</u>—C<u>$H_2$</u>); 1.33 and 1.28 (2t, 6H, 2x-ethyl—C<u>$H_3$</u>, J = 7Hz).

Example 5

7-[N-(2,2-diethoxycarbonyl)vinyl]adipinamido-3-[(1-methyl-1,2,3,4-tetrazol-5-yl)thiomethyl]ceph-3-em-4-carboxylic acid, sodium salt (Process b)

5.85 g of N-[(2,2-diethoxycarbonyl)vinyl]cephalosporin C are mixed with 1.75 g of 5-mercapto-1-methyl-1,2,3,4-tetrazole. The mixture is warmed to 140° and maintained at this temperature for 15 minutes. After cooling, the darkly coloured melt is taken up in 200 ml of acetone, freed by filtration from undissolved material and the filtrate is mixed with a solution of sodiumethylhexoate in acetone (1.7 g) in 30 ml). The resulting precipitate is isolated, washed with acetone and dried at 60° *in vacuo* to obtain the heading compound, m.p. >175° (decomp.).

Example 6

7-[N-(2,2-diethoxycarbonyl)vinyl]adipinamido-3-[(1-methyl-1,2,3,4-tetrazol-5-yl)thiomethyl]ceph-3-em-4-carboxylic acid, sodium salt [Process b)]

5.85 g N-[(2,2-diethoxycarbonyl)vinyl]cephalosporin C together with 1.75 g of 5-mercapto-1-methyl-1,2,3,4-tetrazole are dissolved in 100 ml of 1,1-2-trichloroethane and the solution is brought to boiling. After 50 minutes reflux, the solution is evaporated to remove substantially the solvent, the residue is taken up in 200 ml of acetone and the acetonic solution is worked up as in Example 5 to obtain the heading compound, m.p. >175° (decomp.).

Example 7

7-[N-(2,2-Diethoxycarbonyl)vinyl]adipinamido-3-[(1-methyl-1,2,3,4-tetrazol-5-yl)thiomethyl)-ceph-3-em-4-carboxylic acid sodium salt [(Process b)]

5.85 g N-[(2,2-Diethoxycarbonyl)vinyl]cephalosporin C are mixed well with 2 g of sodium-1-methyl-1,2,3,4-tetrazol-5-thiolate. The powder mixture is then heated for 15 minutes at 140°, whereupon a darkly coloured melt is formed. This is digested with acetone after cooling and the solid material is isolated, washed with acetone and dried *in vacuo* to obtain the heading compound, m.p. >175° (decomp.).

Example 8

7-[N-(2,2-Diethoxycarbonyl)vinyl]adipinamido-3-[(1-methyl-1,2,3,4-tetrazol-5-yl)thiomethyl]-ceph-3-em-4-carboxylic acid, sodium salt [Process b)]

6.07 g of the sodium salt of N-[(2,2-diethoxycarbonyl)]vinyl]-cephalosporin C are mixed well with 1.75 g of 5-mercapto-1-methyl-1,2,3,4-tetrazole and the mixture is treated as in Example 7 to obtain the heading compound, m.p. >175° (decomp.).

Example 9

7-[N-(2,2-Diethoxycarbonyl)vinyl]adipinamido-3-[(benzothiazol-2-yl)thiomethyl]-ceph-3-em-4-carboxylic acid, sodium salt [Process b)]

N-[(2,2-Diethoxycarbonyl)vinyl]cephalosporin C and 2-mercaptobenzothiazole are reacted and worked up in a molecular ratio of 1:1.5 in the same manner as in Examples 5 and 6 to obtain the heading compound, m.p. >180° (decomp.).

Example 10

7-Amino-3-[(1-methyl-1,2,3,4-tetrazol-5-yl)thiomethyl]ceph-3-em-4-carboxylic acid

7 g of 7-[N-(2,2-diethoxycarbonyl)vinyl]adipinamido-3-[(1-methyl-1,2,3,4-tetrazol-5-yl)thiomethyl]-ceph-3-em-4-carboxylic acid sodium salt are suspended in 40 ml of dried methylene dichloride. 11 ml of dimethyl aniline are added at room temperature followed by 10 ml of dimethyldichlorosilane, whereupon the temperature increases to about 33° and a clear solution forms. After 10 minutes, the mixture is cooled to −10° and 3 g of phosphorous pentachloride are added whereupon the temperature rises to about −5°. After 40 minutes stirring at −5°, the reaction solution is poured into 60 ml of pre-cooled methanol (−20°), the temperature then rising to about +5°. After addition of 10 ml of water, the pH value of the solution is adjusted to 3.5 with semi-concentrated ammonia, whereupon the product precipitates out. The precipitate is filtered off, washed with water and methanol and dried *in vacuo* at 50°. The thus obtained light cream powder constitutes the pure heading compound, m.p. >225° (decomp.).

1H—NMR ($D_2O$, $K_2$—$CO_3$): δ = 5.05 (d, 1H, $H_7$, J=5Hz); 4.80 (d, 1H, $H_6$, J=5Hz); 4.20 (q, 2H, $C_3$—$\underline{H}_2$—S—, J=13Hz); 4.05 (s, 3H, N—$C\underline{H}_3$); 3,55 (q, 2H, —$C_2$—$C\underline{H}_2$, J=18Hz).

Example 11

7-Amino-3-[(2,5-dihydro-5-oxo-6-hydroxy-2-methyl-1,2,4-triazin-3-yl)thiomethyl]ceph-3-em-4-carboxylic acid

7 g of 7-[N-(2,2-diethyloxycarbonyl)vinyl]adipinamido-3-[(2,5-dihydro-6-hydroxy-5-oxo-2-methyl-1,2,4-triazin-3-yl)thiomethyl]ceph-3-em-4-carboxylic acid sodium salt are suspended in 40 ml of methylene-dichloride. 1.5 ml of dimethylaniline and 8.8 ml of dimethyldichlorosilane are added, whereupon the temperature rises to 37° and a clear solution forms. After 10 minutes the mixture is cooled to −10° and 2.6 g of phosphorous pentachloride are added, whereupon the temperature increases to −5°. The mixture is stirred at this temperature for 14 minutes and is then poured into 60 ml of pre-cooled dry methanol (−20°), the temperature then increasing to about +5°. 10 ml of water are then added and finally the pH value of the solution is adjusted to 3.5 with semi-concentrated ammonia, whereupon the heading compound precipitates out. The precipitate is filtered, washed with water and methanol and dried *in vacuo* at 50° to obtain the heading compound, m.p. >195° (decomp.).

1H—NMR ($D_2O$, $K_2CO_3$): δ = 5.40 (d, 1H, $H_7$, J=5Hz); 5.00 (d, 1H, $H_6$, J=5Hz); 4.20 (q, 2H, $C_3$—$C\underline{H}_2$—S—, J=13Hz); 3.65 (s, 3H, H—$C\underline{H}_3$); 3.55 (q, 2H, $C_2$—$C\underline{H}_2$—S—, J=18Hz).

Example 12

7-Amino-3-[(1-methyl-1,2,3,4-tetrazol-5-yl)thiomethyl]ceph-3-em-4-carboxylic acid

a) *7-[N-(2,2-Diethoxycarbonyl)vinyl-3-[(1-methyl-1,2,3,4-tetrazol-5-yl)thiomethyl]ceph-3-em-4-carboxylic acid sodium salt*

1 litre of a Cephalosporin C culture filtrate (13 g Cephalosporin C/litre) is reacted with ethoxymethylenemalonic acid ester in the manner described in DOS 3,002,659. The reaction mixture is adjusted to pH 2 and extracted twice with 500 ml of methylisobutylketone. The extract is freed from water by azeotropic distillation *in vacuo* and is evaporated to about 250 ml. 5 g of 1-methyl-5-mercaptotetrazol are added and the mixture is refluxed for 40 minutes. The solvent is removed *in vacuo* and the residue is taken up in 300 ml of acetone and freed from insolubles. The filtrate is mixed with sodium ethylhexoate solution in acetone until precipitation is complete. The precipitate is filtered, washed with acetone and dried *in vacuo* at 50°.

The thus obtained product has a content of about 60% of the heading compound. This crude product can be purified by usual methods (crystallisation, chromatography, etc.) or may be used directly for deacylation.

b) *7-[N-(2,2-Diethoxycarbonyl)vinyl]-3-[(1-methyl-1,2,3,4-tetrazol-5-yl)thiomethyl]ceph-3-em-4-carboxylic acid*

Starting with 1 litre of culture filtrate (Cephalosporin C content = 13 g/l), the method is carried out as described under a) until removal of the methylisobutylketones. The thus obtained oily residue can then either be directly used for deacylation or be digested with ethyl acetate/diisopropylether, whereupon the heading compound is obtained in the form of an amorphous powder of about 50% purity. This can be purified by conventional methods (chromatography, etc.).

c) *7-Amino-3-[(1-methyl-1,2,3,4-tetrazol-5-yl)thiomethyl]ceph-3-em-4-carboxylic acid*
(Deacylation of the crude product obtained under a))

15 g of the crude product obtained under a) (content about 60%) of 7-[N-(2,2-diethoxycarbonyl)vinyl]-3-[(1-methyl-1,2,3,4-tetrazol-5-yl)thiomethyl]ceph-3-em-4-carboxylic acid sodium salt) are suspended in 80 ml of dichloromethane and mixed with 22 ml of dimethylaniline. 20 ml of dimethyldichlorosilane are added with slight cooling and after 15 minutes the resulting clear solution is cooled to −10° and 6 g of phosphorous pentachloride are added. The mixture is stirred for a further 45 minutes at −6° to −10° and then poured into 120 ml of methanol precooled to −20°. After addition of 20 ml of water, the pH value is adjusted to 3.5 by addition of ammonia solution (25%) and the precipitate is filtered off. The precipitate is washed each time with 50 ml of water and methanol and dried at 50° in vacuo to obtain the heading compound as a pure cream product, m.p. >225° (decomp.).

d) *7-Amino-3-[(1-methyl-1,2,3,4-tetrazol-5-yl)thiomethyl]ceph-3-em-4-carboxylic acid*
(Deacylation of the product obtained under b))

The evaporation residue after removal of methylisobutylketones is dissolved in 100 ml of methylenedichloride and mixed with 35 ml dimethylaniline. 25 ml of dimethyldichlorosilane are added with light cooling and the mixture is stirred at room temperature for 15 minutes. The mixture is cooled to −10° and 8 g of phosphorous pentachloride are added. The mixture is stirred for a further 45 minutes at −10° and is poured into 180 ml of methanol pre-cooled to −20°. After addition of 30 ml of water the pH value is adjusted to 3.5 with ammonium hydroxide solution (25%). The precipitate is filtered off, washed with water and methanol and dried at 50° *in vacuo*. The thus-obtained cream product constitutes pure heading compound, m.p. >225° (decomp).

e) *7-Amino-3-[(1-methyl-1,2,3,4-tetrazol-5-yl)thiomethyl]ceph-3-em-4-carboxylic acid*
(Deacylation of pure 7-[N-(2,2-diethoxycarbonyl)vinyl]-3-[(1-methyl-1,2,3,4-tetrazol-5-yl)thiomethyl]ceph-3-em-4-carboxylic acid or its sodium salt)

The method is carried out essentially as described under c) or d) to obtain the heading compound in the form of a cream powder, m.p. >225° (decomp.).
NMR ($D_2O$, $K_2CO_3$): δ = 5.05 (d, 1H, J=5HZ, $H_7$); 4.80 (d, 1H, J=5Hz, $H_6$); 4.20 (q, 2H, J=13Hz, $C_3—C\underline{H}_2—S—$); 4.05 (s, 3H, $N—C\underline{H}_3$); 3.55 (q, 2H, J=18Hz, $C_2—C\underline{H}_2—$).

Example 13

7-Amino-3-[(benzothiazol-2-yl)thiomethyl]ceph-3-em-4-carboxylic acid

21.4 g of 7-[N-(2,2-diethoxycarbonyl)vinyl]adipinamido-3-[(benzothiazol-2-yl)thiomethyl]ceph-3-em-4-carboxylic acid sodium salt are suspended in 120 ml of methylene dichloride and 33 ml of dimethyl aniline are added. 28 ml of dimethyl dichlorosilane are added with slight cooling and the mixture is stirred at room temperature for 15 minutes, whereupon a clear solution results. This is cooled to −10° and 10 g of phosphorous pentachloride are added. After 45 minutes stirring at −6° to −10° the solution is poured in 180 ml of methanol pre-cooled to −20° 30 ml of water are added and the pH value of the solution is adjusted to 3 with 25% ammonium hydroxide. The resulting precipitate is filtered off, washed with water and methanol and dried *in vacuo* at 60°. The resulting product constitutes pure heading compound, m.p. >225°—230° (decomp.).
NMR ($D_2O$, $K_2CO_3$): δ = 7.8—7.0 (m, 4H, Aryl-Protonen); 5.20 (d, 1H, J=5Hz, $Z_7$); 5.00 (d, 1H, J=5Hz, $H_6$); 4.40 (q, 2H, J=13Hz, $C_3—C\underline{H}_2—S—$); 3.45 (q, 2H, J=18Hz, $2—C\underline{H}_2—$).

The necessary N-[(2,2-diethoxycarbonyl)vinyl]cephalosporin C employed as starting material can be obtained in the following manner:

14.2 g of Cephalosporin C monosodium salt dihydrate are dissolved in 30 ml of water. To the resulting mixture, 2.52 g of sodium bicarbonate are added, with stirring, in small portions. After gas evolution has ceased, the residual carbon dioxide is removed from the solution by warming to about 35°. 10 ml of water are added followed by 5.7 g of ethoxymethylenemalonic acid diethyl ester, dissolved in 15 ml of acetone. After 6 hours stirring at room temperature, the acetone is evaporated off. The aqueous phase is extracted with ethyl acetate. The aqueous phase is adjusted to pH 2 and extracted again with ethyl acetate. The ethyl acetate phase is washed with water and dried. After removal of the solvent, the residue is rubbed with

diisopropylether and dried in a desiccator, to obtain the heading compound, m.p. about 80° (decomp.).

**Claims for the Contracting States: BE CH DE FR GB IT LI LU NL SE**

1. A compound of formula

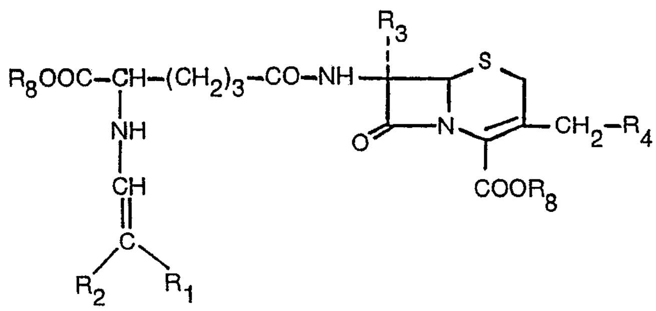

in which $R_4$ is a group $R_6$—S—, in which $R_6$ is a heterocycle, $R_3$ is hydrogen or methoxy, $R_1$ and $R_2$, which may be the same or different, are hydrogen, nitro, cyano, $(C_{1-4})$ alkanoyl or $(C_{1-4})$ alkoxycarbonyl, and the radicals $R_8$, which may be the same or different, each signifies hydrogen or the residue of an ester grouping, or a salt of a compound in which 1 or both of the radicals $R_8$ represent hydrogen.

2. The compound of Claim 1, which is

7-[N-(2,2-diethoxycarbonyl)vinyl]adipinamido-3-[2,5-dihydro-6-hydroxy-5-oxo-2-methyl-1,2,4-triazin-3-yl)thiomethyl]ceph-3-em-4-carboxylic acid;

7-[N-(2,2-diethoxycarbonyl)vinyl]adipinamido-3-[(1-methyl-1,2,3,4-tetrazol-5-yl)thiomethyl]ceph-3-em-4-carboxylic acid;

7-[N-(2,2-diethoxycarbonyl)vinyl]adipinamido-3-[benzothiazol-2-yl)thiomethyl]ceph-3-em-4-carboxylic acid; or a salt thereof.

3. A process for the production of a compound of Claim 1, which comprises reacting a compound of formula Ic,

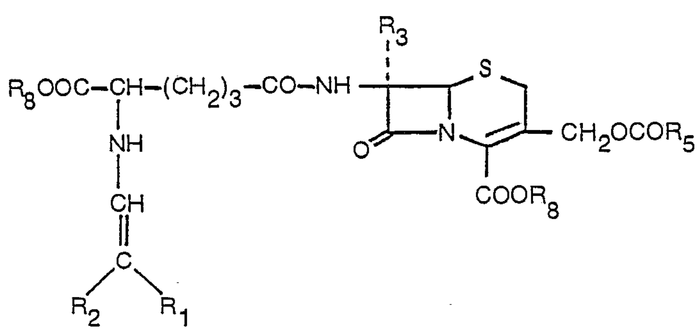

Ic

in which $R_1$, $R_2$, $R_3$ and $R_8$ are as defined in Claim 1, and $R_5$ is $(C_{1-4})$alkyl, amino, mono- or di-$(C_{1-4})$alkyl amino or α-methoxy-β-(p-hydroxyphenyl)vinyl in which the hydroxy group may be esterified with sulphuric acid, with the proviso that $R_1$ and $R_2$ must be independently $(C_{1-4})$alkanoyl when $R_5$ is $CH_3$—, with a compound of formula IV,

$$HS—R_6 \qquad IV$$

in which $R_6$ is as defined in Claim 1.

4. A process for the production of a compound of formula V,

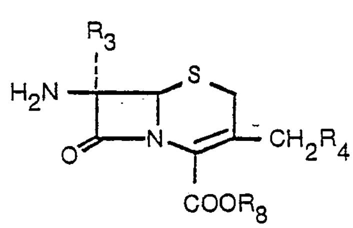

V

in which $R_3$, $R_4$ and $R_8$ are as defined in Claim 1, which comprises deacylating a compound of Claim 1.

5. A process for converting a compound of formula VI,

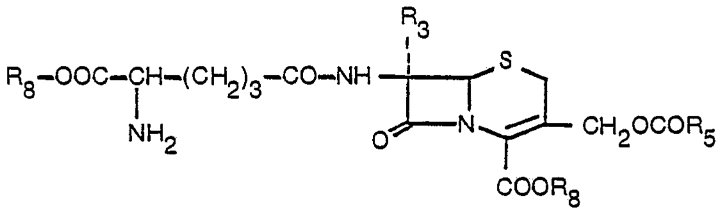

VI

in which $R_3$, $R_5$ and $R_8$ are as defined in Claim 1, into a compound of formula V, stated in Claim 4, and wherein $R_4$ is $R_6$—S— with $R_6$ being as defined in Claim 1, which comprises protecting the free amino group in the 7-aminoadipoyl side chain of the compound of formula VI to obtain a compound of formula Ic as defined in claim 3 wherein $R_1$ and $R_2$ which may be the same or different and each signifies $(C_{1-4})$alkanoyl when $R_5$ is $CH_3$— or hydrogen, nitro, $(C_{1-4})$alkanoyl, cyano, or $(C_{1-4})$alkoxycarbonyl when $R_5$ is other than $CH_3$—, thiolating at the 3-position, and deacylating the resulting protecting compound at the 7-amino group.

**Claims for the Contracting State: AT**

1. A process for the production of a compound of formula I,

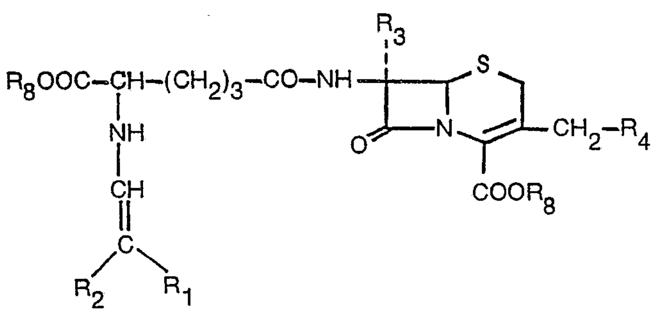

I

in which $R_4$ is a group $R_6$—S—, in which $R_6$ is a heterocycle, $R_3$ is hydrogen or methoxy, $R_1$ and $R_2$, which may be the same or different, are hydrogen, nitro, cyano, $(C_{1-4})$ alkanoyl or $(C_{1-4})$alkoxycarbonyl, and the radicals $R_8$, which may be the same or different, each signifies hydrogen or the residue or an ester grouping, or a salt of a compound in which 1 or both of the radicals $R_8$ represent hydrogen, by reacting a compound of formula IC,

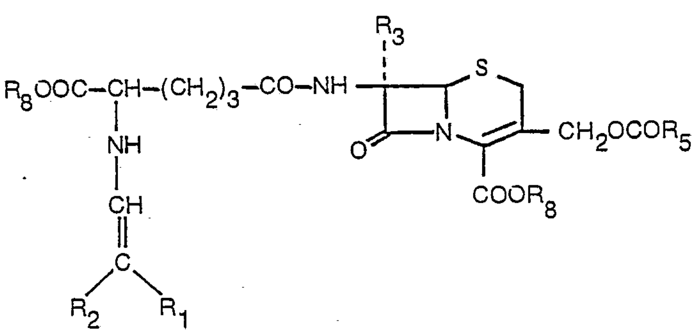

Ic

in which $R_1$, $R_2$, $R_3$ and $R_8$ are as defined above, and $R_5$ is $(C_{1-4})$alkyl, amino, mono- or di-$(C_{1-4})$alkylamino or α-methoxy-β-(p-hydroxy-phenyl)vinyl in which the hydroxy group may be esterified with sulphuric acid and with the proviso that $R_1$ and $R_2$ must be independently $(C_{1-4})$alkanoyl when $R_5$ is $CH_3$—, with a compound of formula IV,

$$HS—R_6 \qquad (IV$$

in which $R_6$ is as defined above.

2. A process for the production of a compound of formula V,

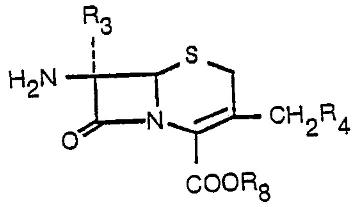

V

in which $R_3$, $R_4$ and $R_8$ are as defined in Claim 1, which comprises deacylating a compound of formula I, stated in Claim 1.

3. A process for converting a compound of formula VI,

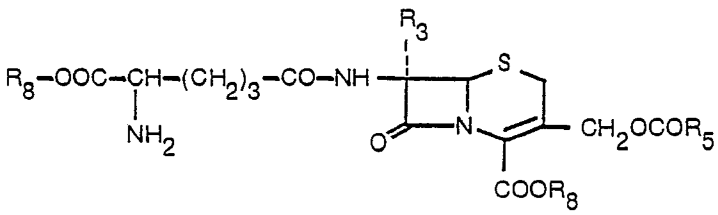

VI

in which $R_3$, $R_5$ and $R_8$ are as defined in Claim 1, into a compound of formula V, stated in Claim 3, and wherein $R_4$ is $R_6$—S with $R_6$ being as defined in Claim 1, which comprises protecting the free amino group in the 7-aminoadipoyl side chain of the compound of formula VI to obtain a compound of formula Ic as defined in Claim 3 wherein $R_1$ and $R_2$ which may be the same or different and each signifies $(C_{1-4})$alkanoyl when $R_5$ is $CH_3$— or hydrogen, nitro, cyano, $(C_{1-4})$alkanoyl or $(C_{1-4})$alkoxycarbonyl when $R_5$ is other than $CH_3$—, thiolating at the 3-position, and deacylating the resulting protecting compound at the 7-amino group.

**Patentansprüche für die Vertragsstaaten: BE CH DE FR GB IT LI LU NL SE**

1. Verbindung der Formel

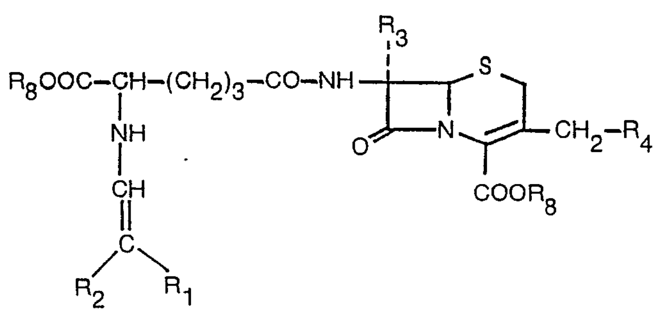

worin $R_4$ für eine $R_6$—S—Gruppe, wobei $R_6$ einen Heterocyclus bedeutet, $R_3$ für Wasserstoff oder die Methoxygruppe stehen, $R_1$ und $R_2$ gleich oder veschieden sein können und Wasserstoff, Nitro, Cyano, $(C_{1-4})$Alkanoyl oder $(C_{1-4})$Alkoxycarbonyl bedeuten, und die Radikale $R_8$, die gleich oder verschieden sein können, für Wasserstoff oder einen Rest einer Estergruppe stehen, oder ein Salz einer Verbindung, worin ein oder beide Radikale $R_8$ Wasserstoff bedeuten.

2. Die Verbindung des Anspruchs 1, die
7-[N-(2,2-Diethoxycarbonyl)vinyl]adipinamido-3-[2,5-dihydro-6-hydoxy-5-oxo-2-methyl-1,2,4-triazin-3-yl)-thiomethyl]ceph-3-em-4-carbonsäure;
7-[N-(2,2-Diethoxycarbonyl)vinyl]adipinamido-3-[(1-methyl-1,2,3,4-tetrazol-5-yl)thiomethyl]ceph-3-em-4-carbonsäure;
7-[N-(2,2-Diethoxycarbonyl)vinyl]adipinamido-3-[benzothiazol-2-yl)thiomethyl]-ceph-3-em-4-carbonsäure ist, oder ein Salz davon.

3. Ein Verfahren zur Herstellung einer Verbindung aus Anspruch 1, dadurch gekennzeichnet, dass man eine Verbindung der Formel Ic

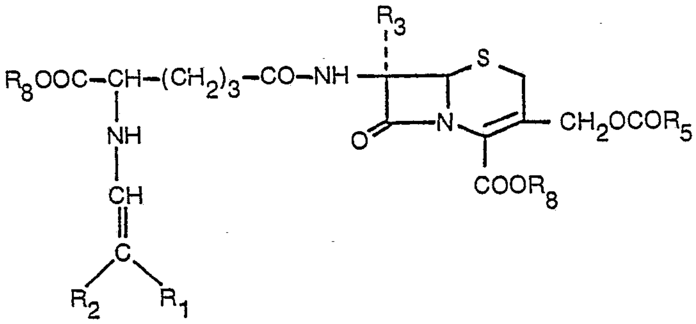

Ic

worin $R_1$, $R_2$, $R_3$ und $R_8$ wie in Anspruch 1 definiert sind und $R_5$ $(C_{1-4})$Alkyl, Amino, Mono- oder Di$(C_{1-4})$alkylamino oder α-Methoxy-β-(p-hydroxyphenyl)vinyl, wobei die Hydroxygruppe durch Schwefelsäure verestert sein kann, bedeutet, mit der Maßgabe, daß $R_1$ und $R_2$ unabhängig $(C_{1-4})$Alkanoyl bedeuten, wenn $R_5$ für $CH_3$ steht, mit einer Verbindung der Formel IV

$$HS—R_6 \qquad IV$$

worin $R_6$ wie in Anspruch 1 definiert ist, umsetzt.

4. Ein Verfahren zur Herstellung einer Verbindung der Formel V

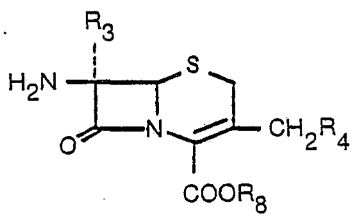

V

worin $R_3$, $R_4$ und $R_8$ wie in Anspruch 1 definiert sind, dadurch gekennzeichnet, daß man eine Verbindung aus Anspruch 1 deacyliert.

5. Ein Verfahren zur Überführung einer Verbindung der Formel VI

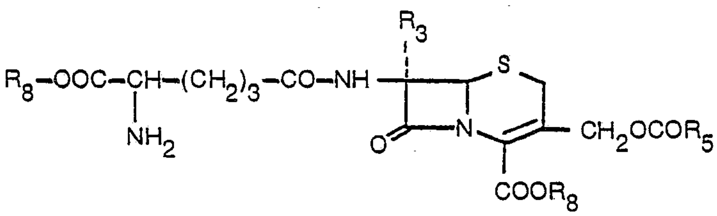

VI

worin $R_3$, $R_5$ und $R_8$ wie in Anspruch 1 definiert sind, in eine Verbindung der Formel V, wie in Anspruch 4 beschrieben, und worin $R_4$ für $R_6$—S—, wobei $R_6$ wie in Anspruch 1 definiert ist, steht, dadurch gekennzeichnet, daß man die freie Aminogruppe in der 7-Aminoadipoylseitenkette der Verbindung der Formel VI schützt, um so eine Verbindung der Formel Ic, wie sie in Anspruch 3 definiert ist, zu erhalten, worin $R_1$ und $R_2$ gleich oder verschieden sein können und jeweils, wenn $R_5$ für $CH_3$ steht, $(C_{1-4})$Alkanoyl oder wenn $R_5$ eine andere Bedeutung als $CH_3$ besitz, Wasserstoff, Nitro, $(C_{1-4})$Alkanoyl, Cyano oder $(C_{1-4})$Alkoxycarbonyl bedeuten, in der 3-Position thioliert und die erhaltene geschützte Verbindung an der 7-Aminogruppe deacyliert.

**Patentansprüche für den Vertragsstaat: AT**

1. Ein Verfahren zur Herstellung einer Verbindung der Formel

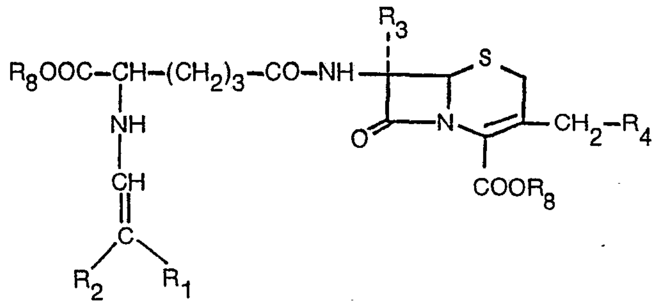

I

worin $R_4$ für eine $R_6$—S—Gruppe, wobei $R_6$ eine Heterocyclus bedeutet, $R_3$ für Wasserstoff oder die Methoxygruppe stehen, $R_1$ und $R_2$ gleich oder verschieden sein können und Wasserstoff, Nitro, Cyano, $(C_{1-4})$Alkanoyl oder $(C_{1-4})$Alkoxycarbonyl bedeuten, und die Radikale $R_8$, die gleich oder verschieden sein können, für Wasserstoff oder einen Rest einer Estergruppe stehen, oder ein Salz einer Verbindung, worin win oder beide Radikale $R_8$ Wasserstoff bedeuten, dadurch gekennzeichnet, daß man eine Verbindung der Formel

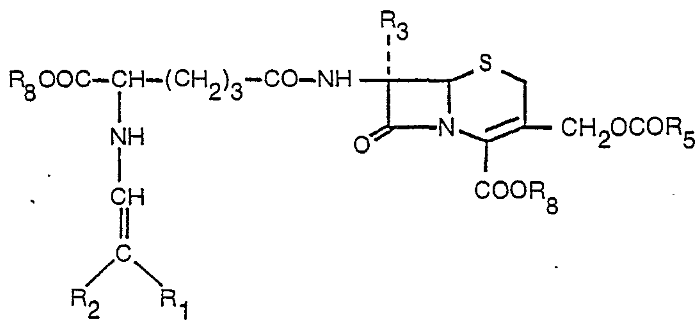

Ic

worin $R_1$, $R_2$, $R_3$ und $R_8$ wie in Anspruch 1 definiert sind und $R_5$ $(C_{1-4})$Alkyl, Amino, Mono- oder Di$(C_{1-4})$alkylamino oder α-Methoxy-β-(p-hydoxyphenyl)vinyl, wobie die Hydroxygruppe durch Schwefelsäure verestert sein kann, bedeutet, mit der Maßgabe, daß $R_1$ und $R_2$ unabhängig $(C_{1-4})$Alkanoyl bedeuten, wenn $R_5$ für $CH_3$ steht, mit einer Verbindung der Formel

$$HS—R_6 \qquad IV$$

worin $R_6$ wie oben definiert ist, umsetzt.

2. Ein Verfahren zur Herstellung einer Verbindung der Formel V

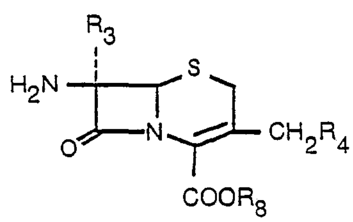

V

worin $R_3$, $R_4$ und $R_8$ wie in Anspruch 1 definiert sind, dadurch gekennzeichnet, daß man eine Verbindung aus Anspruch 1 deacyliert.

3. Ein Verfahren zur Überführung einer Verbindung der Formel VI

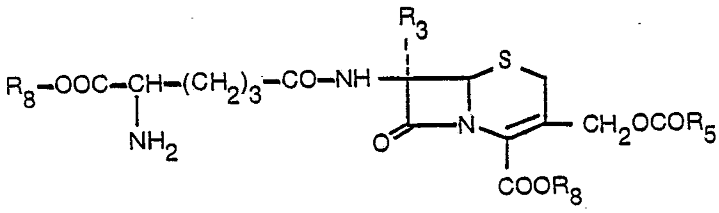

VI

worin $R_3$, $R_5$ und $R_8$ wie in Anspruch 1 definiert sind, in eine Verbindung der Formel V, wie in Anspruch 3 beschrieben, und worin $R_4$ für $R_6$—S—, wobei $R_6$ wie in Anspruch 1 definiert ist, steht, dadurch gekennzeichnet, daß man die freie Aminogruppe in der 7-Aminoadipoylseitenkette der Verbindung der Formel VI schützt, um so eine Verbindung der Formel Ic, wie sie in Anspruch 3 definiert ist, zu erhalten, worin $R_1$ und $R_2$ gleich oder verschieden sein können und jeweils, wenn $R_5$ für $CH_3$ steht, $(C_{1-4})$Alkanoyl oder wenn $R_5$ eine andere Bedeutung als $CH_3$ besitzt, Wasserstoff, Nitro, $(C_{1-4})$Alkanoyl, Cyano oder $(C_{1-4})$Alkoxycarbonyl bedeuten, in der 3-Position thioliert und die erhaltene geschützte Verbindung an der 7-Aminogruppe deacyliert.

**Revendications pour les Etats contractants: BE CH DE FR GB IT LI LU NL SE**

1. Un composé de formule

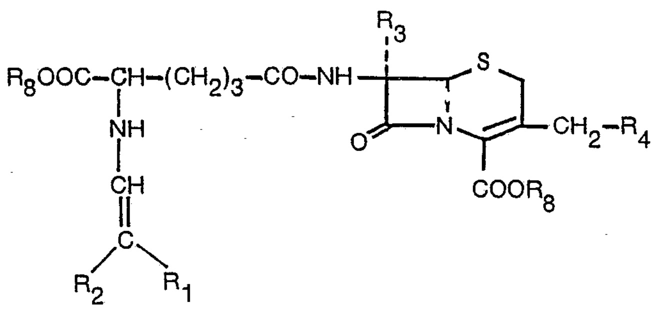

dans laquelle $R_4$ représente un groupe $R_6$—S—, dans lequel $R_6$ est un hétérocycle, $R_3$ représente l'hydrogène ou un groupe méthoxy, $R_1$ et $R_2$, qui peuvent être identiques ou différents, représente l'hydrogène ou un groupe nitro, cyano, alcanoyle en $C_1$—$C_4$ ou alcoxycarbonyle en $C_1$—$C_4$, et les radicaux $R_8$, qui peuvent être identiques ou différents, représentent chacun l'hydrogène ou le reste d'un groupement ester, ou un sel d'un composé dand lequel 1 ou deux des radicaux $R_8$ représentent l'hydrogène.

2. Le composé de la revendication 1, qui est
l'acide 7-[N-(2,2-diéthoxycarbonyl)vinyl]adipinamido-3-[(2,5-dihydro-6-hydroxy-5-oxo-2-méthyl-1,2,4-triazine-3-yl)-thiométhyl]-3-céphème-4-carboxylique;
l'acide 7-[N-(2,2-diéthoxycarbonyl)vinyl]adipinamido-3-[(1-méthyl-1,2,3,4-tétrazole-5-yl)thiométhyl]-3-céphème-4-carboxylique;
l'acide 7-[N-(2,2-diéthoxycarbonyl)vinyl]adipinamido-3-[(benzothiazole-2-yl)thiométhyl]-3-céphème-4-carboxylique; ou un sel de ce composé.

3. Un procédé de préparation d'un composé de formule I, qui comprend la réaction d'un composé de formule Ic

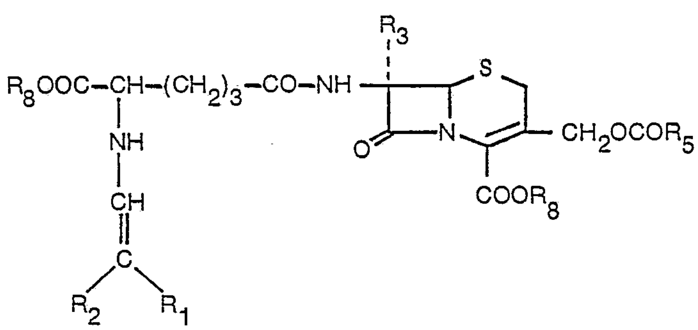

Ic

dans laquelle $R_1$, $R_2$, $R_3$ et $R_8$ sont tels que définis à la revendication 1, et $R_5$ représente un groupe alkyle en $C_1$—$C_4$, amino, mono- ou di-(alkyle en $C_1$—$C_4$)amino ou α-méthoxy-β-(p-hydroxyphényl)vinyle dans lequel le groupe hydroxy peut être estérifié avec l'acide sulfurique, avec la condition que $R_1$ et $R_2$ doivent signifier indépendamment un groupe alcanoyle en $C_1$—$C_4$ lorsque $R_5$ signifie $CH_3$—, avec un composé de formule IV

$HS—R_6$ (IV)

dans laquelle $R_6$ est tel que défini à la revendication 1.

4. Un procédé de préparation d'un composé de formule V

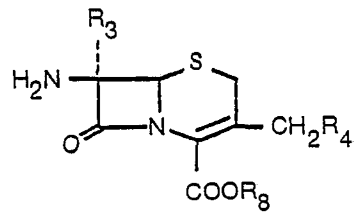

V

dans laquelle $R_3$, $R_4$ et $R_8$ sont tels que définis à la revendication 1, qui comprend la désacylation d'un composé de la revendication 1.

5. Un procédé de transformation d'un composé de formule VI

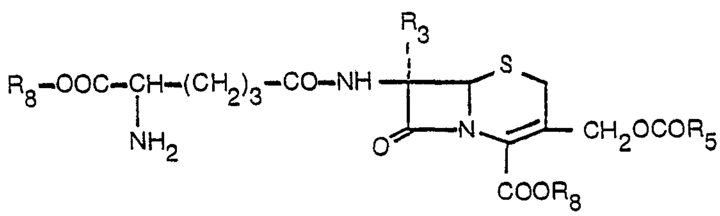

VI

dans laquelle $R_3$, $R_5$ et $R_8$ sont tels que définis à la revendication 1, en un composé de formule V, spécifié à la revendication 4, et dans laquelle $R_4$ signifie $R_6—S—$, où $R_6$ est tel que défini à la revendication 1, qui comprend la protection du groupe amino libre dans la chaîne latérale 7-aminoadipoyle du composé de formule VI pour obtenir un composé de formule Ic tel que défini à la revendication 3 dans laquelle $R_1$ et $R_2$, qui peuvent être identiques ou différents, signifient chacun un groupe alcanoyle en $C_1—C_4$ lorsque $R_5$ signifie $CH_3—$ ou l'hydrogène ou un groupe nitro, alcanoyle en $C_1—C_4$, cyano ou alcoxycarbonyle en $C_1—C_4$ lorsque $R_5$ est autre que $CH_3—$, la thiolation en position 3 et la déacylation du composé protégé résultant sur le groupe 7-amino.

**Revendications pour l'Etat contractant: AT**

1. Un procédé de préparation d'un composé de formule I

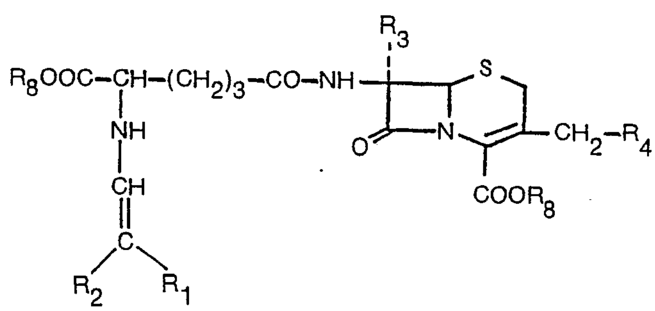

I

dans laquelle $R_4$ représente un groupe $R_6—S—$, dans lequel $R_6$ est un hétérocycle, $R_3$ représente l'hydrogène ou un groupe méthoxy, $R_1$ et $R_2$, qui peuvent être identiques ou différents, représentent l'hydrogène ou un groupe nitro, cyano, alcanoyle en $C_1—C_4$ ou alcoxycarbonyle en $C_1—C_4$ et les radicaux $R_8$, qui peuvent être identiques ou différents, signifient chacun l'hydrogène ou le reste d'un groupement ester, ou un sel d'un composé dans lequel 1 ou deux des radicaux $R_8$ représentent l'hydrogène, par réaction d'un composé de formule Ic

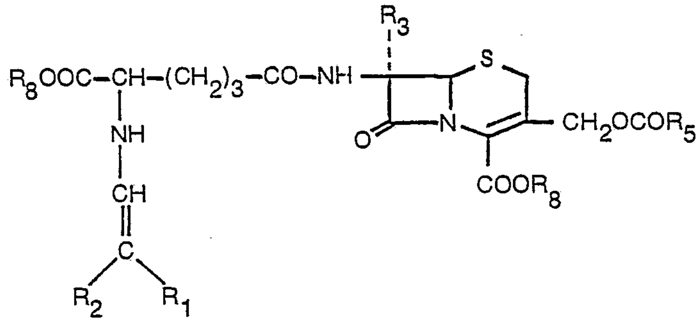

Ic

dans laquelle $R_1$, $R_2$, $R_3$ et $R_8$ sont tels que définis ci-dessus et $R_5$ représente un groupe alkyle en $C_1$—$C_4$, amino, mono- ou di-(alkyle en $C_1$—$C_4$)amino ou α-méthoxy-β-(p-hydroxy-phényl)vinyle dans lequel le groupe hydroxy peut être estérifié avec l'acide sulfurique et avec la condition que $R_1$ et $R_2$ doivent signifier indépendamment un groupe alcanoyle en $C_1$—$C_4$ lorsque $R_5$ signifie $CH_3$—, avec un composé de formule IV

$$HS—R_6 \qquad (IV)$$

dans laquelle $R_6$ est tel que défini ci-dessus.

2. Un procédé de préparation d'un composé de formule V

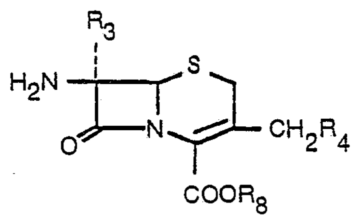

V

dans laquelle $R_3$, $R_4$ et $R_8$ sont tels que définis à la revendication 1, qui comprend la désacylation d'un composé de formule I, spécifié à la revendication 1.

3. Un procédé de transformation d'un composé de formule VI

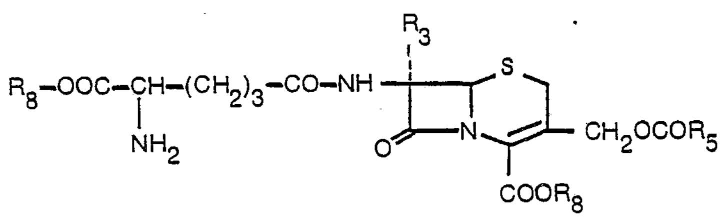

VI

dans laquelle $R_3$, $R_5$ et $R_8$ sont tels que définis à la revendication 1, en un composé de formule V, spécifié à la revendication, et dans laquelle $R_4$ signifie $R_6$—S où $R_6$ est tel que défini à la revendication 1, qui comprend la protection du groupe amino libre dans la chaîne latérale 7-aminoadipoyle du composé de formule VI pour obtenir un composé de formule Ic tel que défini à la revendication 3 dans laquelle $R_1$ et $R_2$, qui peuvent être identiques ou différents, signifient chacun un groupe alcanoyle en $C_1$—$C_4$ lorsque $R_5$ signifie $CH_3$— ou l'hydrogène ou un groupe nitro, cyano, alcanoyle en $C_1$—$C_4$ ou alcoxycarbonyle en $C_1$—$C_4$ lorsque $R_5$ est autre que $CH_3$—, la thiolation en position 3 et la désacylation du composé protégé résultant sur le groupe 7-amino.